# EUROPEAN PATENT APPLICATION

(11) **EP 3 777 781 A1**
(43) Date of publication of application: **17.02.2021**
(21) Application number: 19774965.8
(22) Date of filing: 14.03.2019
(51) Int. Cl.: A61F 2/88, A61F 2/90, A61F 2/95

(54) **STENT**

(30) Priority: 30.03.2018 JP 2018068342
(71) Applicant: KURUME UNIVERSITY, Kurume-shi Fukuoka 830-0011 (JP); Medikit Co., Ltd., Tokyo, 113-0034 (JP); TOKUSEN KOGYO CO., LTD., Ono-city Hyogo 675-1361 (JP)
(72) Inventor: FUKUMOTO, Yoshihiro, Kurume-shi, Fukuoka 830-0011 (JP); TANAKA, Yasuomi, Tokyo 113-0034 (JP); UMEDA, Masahiro, Tokyo 113-0034 (JP); NAM, Kwangwoo, OnoCity, Hyogo 6751361 (JP)
(74) Representative: Grünecker Patent- und Rechtsanwälte PartG mbB
(86) International application number: PCT/JP2019/010562
(87) International publication number: WO 2019/188345

(57) **Abstract**

Provided is a stent that can sufficiently support the inner wall of an in vivo lumen from the inside, and that can be easily collected outside the body after the stent completed its role. The stent 1 is delivered from the dissected base of a patient's leg through the inside of a catheter 2 that is a hollow flexible tube. The stent 1 is a coil (support) member that supports the inner wall of an in vivo lumen from the inside while being placed at a treated area in the in vivo lumen. The stent 1 takes an elongate form to elongate along the inside of the catheter 2 by pulling the stent 1 in a longitudinal direction and a coil form to support the inner wall of an in vivo lumen from the inside by releasing the stent 1. The stent 1 also has a hook 1b for collection at its one end.

## Description

### TECHNICAL FIELD

The present invention relates to a stent. More specifically, the present invention relates to a stent for medical treatment, which is used to treat an in vivo lumen.

### BACKGROUND

The aortic dissection causes sudden death. The patients with aortic dissection mostly need to undergo emergency surgery when they are taken to a hospital. Since the surgical technique for aortic dissection is highly difficult in urgent situations, the physicians and the patients face extreme stress. As a method for treating aortic dissection and other disease, the method of placing a metallic stent in an in vivo lumen such as a blood vessel is conventionally known (refer to Patent Document 1, for example). The treatment with a stent has a less-invasive advantage because an abdominal surgery is unnecessary. However, a stent that has been placed in a blood vessel for a long time may form a blood clot to cause thrombotic occlusion. Even after the treated area has been cured, the stent that completed its role and remains in the body as foreign body may cause foreign body response.

Then, as disclosed in Patent Document 2, for example, a bioabsorbable stent that is melted away a few years after it is placed in the body has been proposed.

### Citation List:

### Patent Literature

Patent Document 1: JP 2008-514370 A
Patent Document 2: JP 2011-206114 A

### SUMMARY

However, a bioabsorbable stent has the problem of the strength and the thickness, compared with a metallic stent. A thicker bioabsorbable stent keeps stronger but easily forms a blood clot. On the other hand, a thinner bioabsorbable stent become weaker to possibly break and cannot sufficiently support the inner wall of an in vivo lumen from the inside.

An objective of the present invention is to provide a stent that is the most appropriate for emergency treatment for aortic dissection and other diseases, that can sufficiently support the inner wall of an in vivo lumen from the inside, and that can be easily collected outside the body.

According to the first aspect of the present invention, a stent that is delivered through the inside of a flexible hollow tube and supporting the inner wall of an in vivo lumen from the inside while the stent is being placed in the in vivo lumen, includes:
a structure that takes:
   an elongate form to elongate along the inside of the flexible tube by pulling the stent in a longitudinal direction, and
   a support form to support the inner wall of the in vivo lumen from the inside by releasing the stent; and
a hook for collection, in which
the stent moves backward through the inside of the flexible tube, transforming itself from the support form to the elongate form, by hooking the hook on a snare for collection and pulling the hook in the flexible tube, and then the stent is collected outside the body.

The stent according to the first aspect of the present invention has the following function effect. Since it takes an elongate form to elongate along the inside of the flexible tube by pulling the stent in a longitudinal direction, the stent easily passes through the inside of a flexible tube and then is easily delivered to a treated area. Since it takes a support form to support the inner wall of an in vivo lumen from the inside after it is delivered and released, the stent can play its primary role, for example, to support the inner wall of a blood vessel from the inside. Since it takes an elongate form to elongate along the inside of the flexible tube by pulling the stent in a longitudinal direction, the stent that supports the inner wall of an in vivo lumen from the inside while being placed in the in vivo lumen can be easily collected outside the body by hooking the hook on a snare for collection and pulling the hook in the flexible tube. Thus, according to the firs aspect of the present invention, the stent can sufficiently support the inner wall of an in vivo lumen from the inside and can be easily collected outside the body.

The stent according to the first aspect of the present invention preferably has the below-mentioned configuration according to any one of the second to twentieth aspects of the present invention.

According to the second aspect of the present invention, the hook is formed at one end of the stent, and the stent is coiled from the hook while maintaining the support form. According to the preferable configuration of the second aspect of the present invention, the stent can approximately evenly support the inner wall of an in vivo lumen from the inside.

According to the third aspect of the present invention, the hook is formed at one end of the stent, and the stent while maintaining the support form includes:
a first helical part that elongates in a first helical form from the hook;
a turned-back part that is formed at the end of the first helical part that is opposite to the hook; and
a second helical part that elongates in a second helical form from the turned-back part and connects to the hook, the second helical form that is coiled in an opposite direction to the first helical form. According to the preferable configuration of t the third aspect of the present invention, the stent can more evenly support the inner wall of an in vivo lumen from the inside by the two helical parts.

According to the fourth aspect of the present invention, the hook is formed at one end of the stent, and the stent while maintaining the support form includes:
a first pulse part that elongates in a first pulse form from the hook in a longitudinal direction, the first pulse part that is approximately semicircular, viewed from the end in a longitudinal direction;
a turned-back part that is formed at the end of the first pulse part that is opposite to the hook; and
a second pulse part that elongates in a second pulse form from the turned-back part toward the hook and connects to the hook, the second pulse form that is plane-symmetrical against the first pulse form. According to the preferable configuration of the fourth aspect of the present invention, the stent hardly falls over sideways in a longitudinal direction when or after it is placed at a treated area in an in vivo lumen. Moreover, the stent hardly slides and moves over in an in vivo lumen because the wire forming the stent does not run in one direction. In addition, the stent hardly crashes under pressure from the outer periphery because the semicircular parts of the wire forming the stent elongate in a circumferential direction and are parallel one after another. Therefore, the stent can firmly support the inner wall of an in vivo lumen from the inside.

According to the fifth aspect of the present invention, in the stent according to the fourth aspect of the present invention, the pulse parts adjacent in a circumferential direction are interlaced and engaged with each other (interlacing engagement). According to preferable configuration of the fifth aspect of the present invention, the stent can prevent the two pulse parts from moving over in a longitudinal direction oppositely to each other to maintain its cylindrical form. Therefore, the stent can more firmly support the inner wall of an in vivo lumen from the inside. The stent can tightly support the inner wall of an in vivo lumen from the inside because the outside diameter of the stent can conform to the inside diameter of an in vivo lumen by not welding but interlacing and engaging the pulse parts adjacent in a circumferential direction with each other. Particularly when the inside diameter of an in vivo lumen is smaller than the outside diameter of the stent, the interlaced and engaged parts are biting into the inner wall of an in vivo lumen to put tension on the stent. Therefore, the stent can tightly support the inner wall of an in vivo lumen from the inside.

According to the sixth aspect of the present invention, the hook is formed at one end of the stent, and the stent while maintaining the support form includes:
a first zig-zag part that elongates in a first zig-zag form from the hook in a longitudinal direction, the first zig-zag part that is approximately semicircular, viewed from the end in a longitudinal direction;
a turned-back part that is formed at the end of the first zig-zag part that is opposite to the hook; and
a second zig-zag part that elongates in a second zig-zag form from the turned-back part toward the hook and connects to the hook, the second zig-zag form that is plane-symmetrical against the first zig-zag form. According to the preferable configuration of the sixth aspect of the present invention, the stent more hardly falls over sideways in a longitudinal direction when or after it is placed at a treated area in an in vivo lumen, compared with the coiled stent. Moreover, the stent hardly slides and moves over in an in vivo lumen because the wire forming the stent does not run in one direction. In addition, since it elongates in a zig-zag form in a longitudinal direction, the stent more hardly falls over sideways, compared with the stent according to the fourth aspect of the present invention. Therefore, the stent can firmly support the inner wall of an in vivo lumen from the inside.

According to the seventh aspect of the present invention, in the stent according to the sixth aspect of the present invention, the adjacent zig-zag parts are interlaced and engaged with each other in a circumferential direction (interlacing engagement). According to the preferable configuration of the seventh aspect of the present invention, the stent can prevent the two zig-zag parts from moving over in a longitudinal direction oppositely to each other to maintain its cylindrical form. Therefore, the stent can more firmly support the inner wall of an in vivo lumen from the inside. The stent can tightly support the inner wall of an in vivo lumen from the inside because the outside diameter of the stent can conform to the inside diameter of an in vivo lumen by the adjacent zig-zag parts interlaced and engaged with each other in a circumferential direction. Particularly when the inside diameter of an in vivo lumen is smaller than the outside diameter of the stent, the interlaced and engaged parts are biting into the inner wall of an in vivo lumen to put tension on the stent. Therefore, the stent can tightly support the inner wall of an in vivo lumen from the inside.

According to the eighth aspect of the present invention, the stent is formed of a shape-memory alloy wire, and includes a structure that takes an elongate form to elongate along the inside of the flexible tube when the stent is cooled below a body temperature, and a support form to support the inner wall of an in vivo lumen from the inside when the stent is warmed to a body temperature. According to the preferable configuration of the eighth aspect of the present invention, since it is formed of a shape-memory alloy, the stent can maintain sufficient strength even if the thickness is reduced. Therefore, the thinner stent hardly forms a blood clot. Moreover, since it takes an elongate form to elongate along the inside of the flexible tube when the stent is cooled below a body temperature, the stent easily passes through the inside of the flexible tube and is easily delivered to a treated area. Moreover, since it takes a support form to support the inner wall of an in vivo lumen from the inside when the stent is warmed to a body temperature after it is delivered to a treated area, the stent can play its primary role, for example, to support the inner wall of a blood vessel from the inside. Since it takes an elongate form to elongate along the inside of the flexible tube by cooling below a body temperature, the stent that supports the inner wall of an in vivo lumen from the inside of the in vivo lumen while it is being placed in the in vivo lumen can be easily collected outside the body by hooking the hook on a snare for collection and pulling the hook in the flexible tube.

According to the ninth aspect of the present invention, the hook is located radially inside from the outer periphery of the stent when it takes the support form. According to the preferable configuration of the ninth aspect of the present invention, since it is located in a radially inner direction from the outer periphery of the stent when the stent takes the support form, the hook does not damage the inner wall of an in vivo lumen during treatment.

According to the tenth aspect of the present invention, the outside diameter of the stent when it takes the support form is from 30 to 55 mm, approximately same as the inside diameter of the aorta that is an in vivo lumen. According to the preferable configuration of the tenth aspect of the present invention, the stent can put an appropriate tension to the inner wall of the blood vessel when it is placed in the aorta to treat aortic dissection. No tension is excessively put, so that the recovery of the entry part of aortic dissection can be properly promoted.

According to the eleventh aspect of the present invention, in the stent according to tenth aspect of the present invention, the outside diameter of the stent changes in a longitudinal direction when it takes the support form. According to the preferable configuration of the eleventh aspect of the present invention, appropriate treatment can be offered because the outside diameter of the stent changes with changes in the inside diameter of the stenting site even if the inside diameter of the stenting site changes in a longitudinal direction. For example, the stent can firmly support an in vivo lumen when the inside diameter of an in vivo lumen is partially increased. Moreover, the stent does not put an excessive tension to an in vivo lumen even if the inside diameter of the in vivo lumen is partially decreased.

According to the twelfth aspect of the present invention, in the stent according to any one of the eighth to the eleventh aspect of the present invention, the diameter of the wire is from 0.3 to 0.7 mm. According to the preferable configuration of the twelfth aspect of the present invention, the stent can efficiently control the formation of a blood clot while maintaining its strength.

According to the thirteenth aspect of the present invention, in the stent according to any one of the eighth to the twelfth aspects of the present invention, the tensile strength of the wire is from 900 to 1500 MPa. According to the preferable configuration of the thirteenth aspect of the present invention, the stent can put an appropriate tension to the inner wall of an in vivo lumen while maintaining its strength.

According to the fourteenth aspect of the present invention, in the stent according to any one of the eighth to the thirteenth aspects of the present invention, the material of the wire is any one of a nickel-titanium alloy, a stainless steel, titanium, and a titanium alloy. According to the preferable configuration of the fourteenth aspect of the present invention, the stent can be achieved by meeting the required biocompatibility and hardly making itself deformed, for example, crashed or folded even if it passes through the inside of a thin flexible tube.

According to the fifteenth aspect of the present invention, in the stent according to any one of the eighth to the fourteenth aspects of the present invention, the wire has a resin layer on the surface. According to the preferable configuration of the fifteenth aspect of the present invention, the stent can improve its biocompatibility. Moreover, the stent can be appropriately frictioned against the inner wall of an in vivo lumen while it is delivered. In addition, the stent can appropriately adjust its pitches in a longitudinal direction on stenting. Therefore, the stent can sufficiently support the inner wall of an in vivo lumen from the inside.

According to the sixteenth aspect of the present invention, in the stent according to the fifteenth aspect of the present invention, the thickness of the resin layer is from 0.01 to 3.00 mm. The resin layer with a thickness of 0.01 mm or more can reduce the possibility of damage to the stent because the thickness is not too thin. The resin layer with a thickness of 3.00 mm or less can efficiently control the formation of a blood clot because the thickness is not too thick.

According to the seventeenth aspect of the present invention, in the stent according to the fifteenth or the sixteenth aspect of the present invention, the material of the resin layer is any one of PU (polyurethane), PA (polyamide), PP (polypropylene), PE (polyethylene), and a fluorine resin. According to the preferable configuration of the seventeenth aspect of the present invention, since it has biocompatibility, the stent is not corroded to minimize the side effect to a human body after it has been inserted in an in vivo lumen.

According to the eighteenth aspect of the present invention, the surface of the stent is coated with a medicine. According to the preferable configuration of the eighteenth aspect of the present invention, the stent can effectively promote the recovery of the treated area after stenting.

According to the nineteenth aspect of the present invention, in the stent according to the eighteenth aspect of the present invention, the medicine is a physiologically active substance.

According to the twentieth aspect of the present invention, the stent is used to treat aortic dissection.

According to the present invention, the stent can sufficiently support the inner wall of an in vivo lumen from the inside and can be easily collected outside the body.

### BRIEF DESCRIPTION OF DRAWINGS

Fig. 1 is a perspective view illustrating the configuration of the stent of Embodiment 1 of the present invention.
Fig. 2 is a side view illustrating the configuration of the stent of Embodiment 1 of the present invention.
Fig. 3 is an end view illustrating the configuration of the stent of Embodiment 1 of the present invention, viewed from the arrow A of Fig. 1.
Fig. 4 illustrates to explain aortic dissection and the method of treating the same.
Fig. 5 is an exploded side view illustrating a catheter introducer used to place the stent of Embodiment 1 of the present invention at a treated area in an in vivo lumen.
Fig. 6 is a side view illustrating a catheter introducer used to place the stent of Embodiment 1 of the present invention at a treated area in an in vivo lumen.
Fig. 7 is a side perspective view illustrating the state in which the tip of a catheter having reached near a treated area in an in vivo lumen.
Fig. 8 is a side perspective view illustrating the state in which the stent of Embodiment 1 of the present invention is being brought to the tip of a catheter by using a clamp for stent insertion.
Fig. 9 is a side perspective view illustrating the state in which the stent of Embodiment 1 of the present invention has been placed at a treated area in an in vivo lumen.
Fig. 10 is a side perspective view illustrating the state in which the catheter introducer is being removed after the stent of Embodiment 1 of the present invention has been placed at the treated area in an in vivo lumen.
Fig. 11 is a side perspective view illustrating the configuration of a snare for collection that is used to collect the stent of Embodiment 1 of the present invention.
Fig. 12 is a side perspective view illustrating the state in which the operation wire that is a structural member of the snare for collection of Fig. 11 has been inserted movably forward and backward inside a catheter that is a hollow flexible tube.
Fig. 13 is a side perspective view illustrating the state in which the tip of a catheter that is a hollow flexible tube has reached near a treated area after the snare for collection of Fig. 11 was placed in the catheter.
Fig. 14 is a side perspective view illustrating the state in which the snare wire that is a structural member of the snare for collection of Fig. 11 has been pushed out of the tip of the catheter in the state of Fig. 13.
Fig. 15 is a side perspective view illustrating the state in which the snare wire that is a structural member of the snare for collection of Fig. 11 has been hooked on the stent of Embodiment 1 of the present invention.
Fig. 16 enlarges the part I enclosed by the chain double-dashed line shown in Fig. 15.
Fig. 17 is a side perspective view illustrating the state in which the stent of Embodiment 1 of the present invention is being pulled in a catheter that is a hollow flexible tube with the snare for collection of Fig. 11.
Fig. 18 is a side perspective view illustrating the state in which a catheter that is a hollow flexible tube is being removed after the stent of Embodiment 1 of the present invention was pulled in the catheter with the snare for collection of Fig. 11.
Fig. 19 is a perspective view illustrating other configurations of the stent of Embodiment 1 of the present invention.
Fig. 20 is a perspective view illustrating the configuration of the stent of Embodiment 2 of the present invention.
Fig. 21 is a perspective view illustrating the configuration of the stent of Embodiment 3 of the present invention.
Fig. 22 includes the pattern diagrams (a) to (d) illustrating the pulse parts of the stent of Embodiment 3 of the present invention, the side view (e) illustrating the configuration of the stent of Embodiment 3 of the present invention, and the diagram (f) of the stent of Embodiment 3 viewed from the end in a longitudinal direction.
Fig. 23 is a perspective view illustrating the configuration of the stent of Embodiment 4 of the present invention.
Fig. 24 is a perspective view illustrating the configuration of the stent of Embodiment 5 of the present invention.
Fig. 25 includes the pattern diagrams (a) to (d) illustrating the zig-zag parts of the stent of Embodiment 5 of the present invention, the side view (e) illustrating the configuration of the stent of Embodiment 5 of the present invention, and the diagram (f) of the stent of Embodiment 5 viewed from the end in a longitudinal direction.
Fig. 26 is a perspective view illustrating the configuration of the stent of Embodiment 6 of the present invention.

### DETAILED DESCRIPTION

The present invention will be more specifically described below with reference to the preferable embodiments. However, these are illustrative only, and the present invention is not limited thereto.

### Embodiment 1

### Configuration of stent

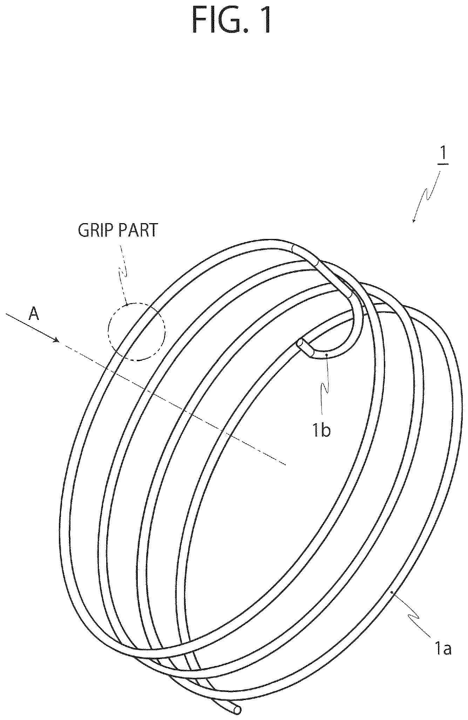

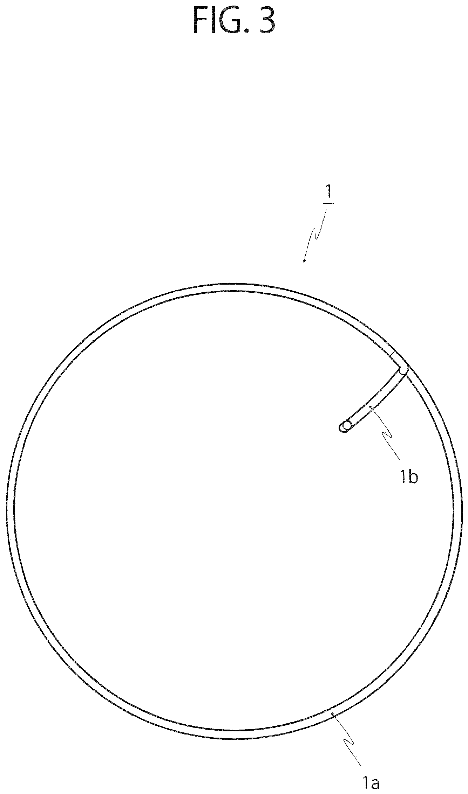

The configuration of the stent of Embodiment 1 of the present invention is described below with reference to Figs. 1 to 3.

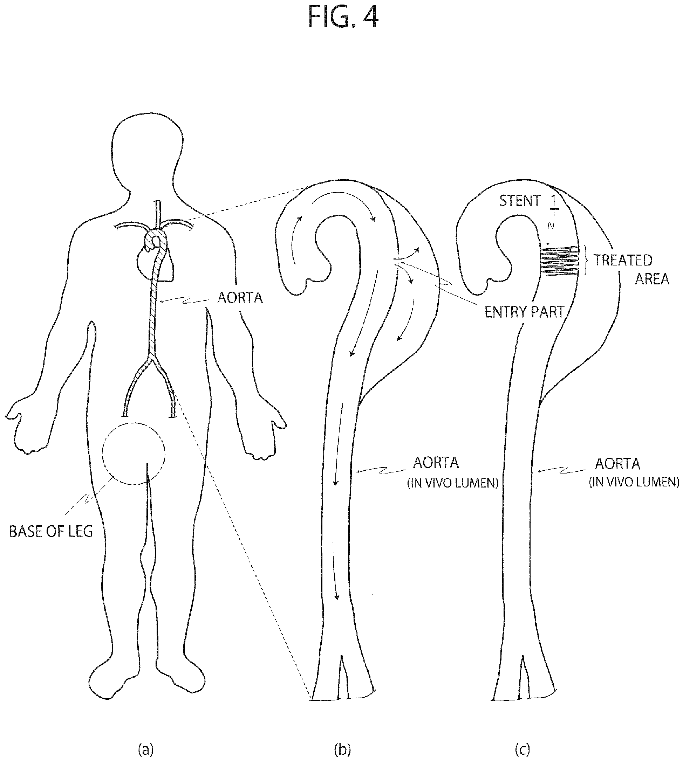

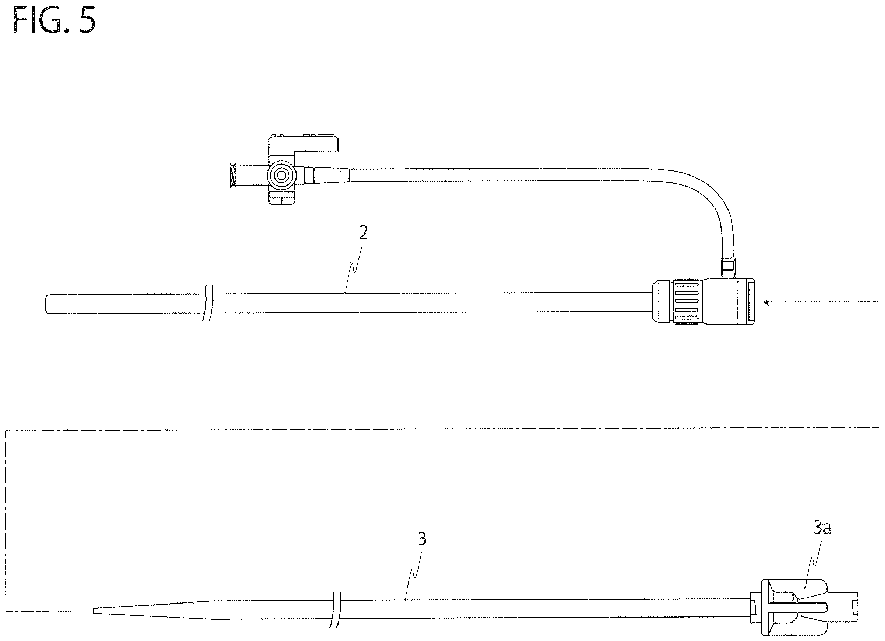

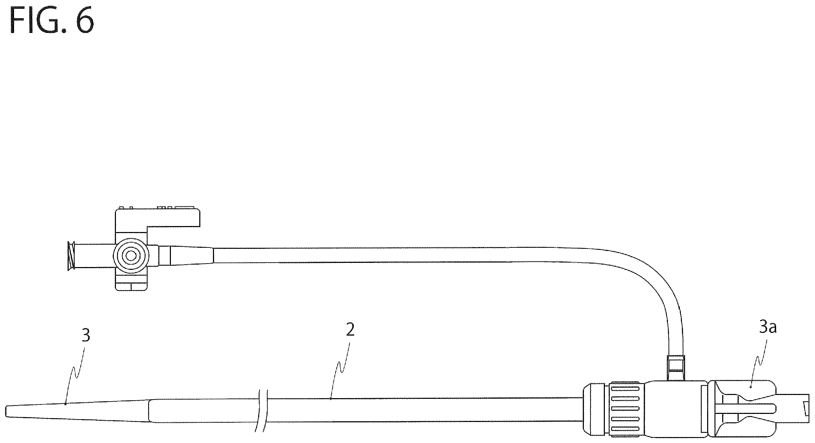

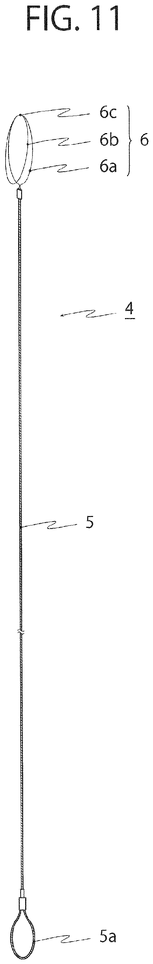

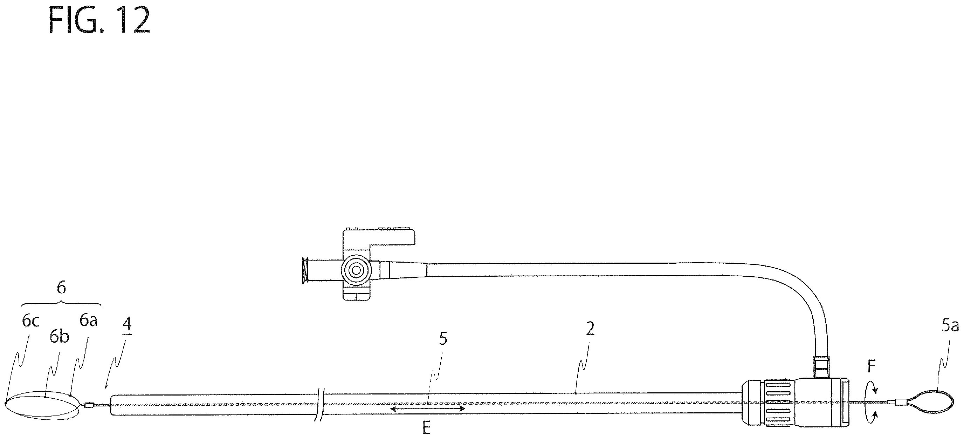

The stent 1 shown in Figs. 1 to 3 is delivered from, for example, the dissected base of a patient's leg through the inside of a catheter 2 (refer to Figs. 5 and 6) that is a hollow flexible tube. The stent 1 is a coiled (helical) member that supports the inner wall of an in vivo lumen from the inside while being placed at a treated area in the in vivo lumen (refer to Fig. 4). The stent 1 takes an elongate form to elongate along the inside of the catheter 2 by pulling the stent 1 in a longitudinal direction and a coil (support) form to support the inner wall of an in vivo lumen from the inside by releasing the tension in a longitudinal direction. The stent 1 also has a hook 1b for collection at its one end. Specifically, the stent 1 of this embodiment has a hook 1b formed at its one end and is coiled from the hook 1b while maintaining the support form. The hook 1b may be located at the leading end or the base end of the stent 1. The stent 1 moves backward through the inside of the catheter 2, transforming itself from the coil form to the elongate form, by hooking the hook 1b on a snare for collection 4 (refer to Figs. 11 and 12) and pulling the hook 1b in the catheter 2, and then can be collected outside the body.

According to the stent 1 of this embodiment, since it takes an elongate form to elongate along the inside of the catheter 2 by pulling the stent 1 in a longitudinal direction, the stent easily passes through the inside of a catheter 2 and then is easily delivered to a treated area. Since it takes a coil (support) form to support the inner wall of an in vivo lumen from the inside after it is delivered and released, the stent can play its primary role, for example, to support the inner wall of a blood vessel from the inside. Since the support form is a coil form, the stent 1 can approximately evenly support the inner wall of an in vivo lumen from the inside. Since it takes an elongate form to elongate along the inside of the catheter 2 by pulling the stent 1 in a longitudinal direction, the coiled stent 1 that supports the inner wall of an in vivo lumen from the inside while being placed in the in vivo lumen can be easily collected outside the body by hooking the hook 1b on a snare for collection 4 and pulling the hook 1b in the catheter 2. Thus, according to the stent 1 of this embodiment, the stent can sufficiently support the inner wall of an in vivo lumen from the inside and can be easily collected outside the body.

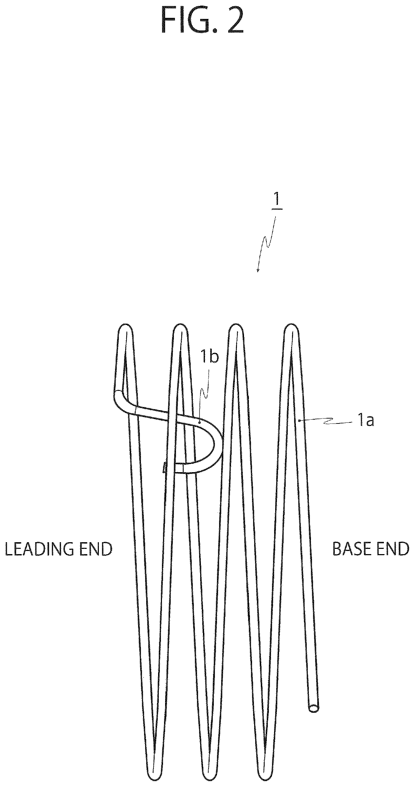

The configuration of the stent of Embodiment 1 of the present invention is more specifically described below. As shown in Figs. 1 to 3, the stent 1 has a coiled stent main body 1a and a hook 1b at one end of the stent main body 1a. The hook 1b is formed by bending of one end of the coil. The hook 1b is located radially inside from the outer periphery of the stent main body 1a when the stent main body 1a takes a coil form to support the inner wall of an in vivo lumen from the inside. Accordingly, the hook 1b does not damage the inner wall of an in vivo lumen during treatment. As shown in Fig. 2, the hook 1b is formed at the leading end of the coiled stent main body 1a and elongates from the leading end toward the base end and projects in a diameter direction. The "grip part" shown in Fig. 1 is to be gripped with a clamp for stent insertion that will be described later when the stent 1 is placed at a treated area in an in vivo lumen. The position of the grip part is not limited to the position shown in Fig. 1 but only has to be in the leading end side of the stent 1. For example, the hook 1b may be a grip part.

The stent 1 is formed of one shape-memory alloy wire with a circular cross-section. The stent 1 takes an elongate form to elongate along the inside of a catheter 2 when the stent 1 is cooled below a body temperature and a coil form to support the inner wall of an in vivo lumen from the inside when the stent 1 is warmed to a body temperature. Accordingly, since it is formed of a shape-memory alloy, the stent 1 can maintain sufficient strength even if the thickness is reduced. Therefore, the thinner stent hardly forms a blood clot. Moreover, since it takes an elongate form to elongate along the inside of the catheter 2 when the stent 1 is cooled below a body temperature, the stent 1 passes through the inside of the catheter 2 and is delivered to a treated area, easily. Moreover, since it takes a coil form to support the inner wall of an in vivo lumen from the inside when the stent 1 is warmed to a body temperature after it is delivered to a treated area, the stent 1 can play its primary role, for example, to support the inner wall of a blood vessel from the inside. Moreover, the stent 1 takes an elongate form to elongate along the inside of the catheter 2 when it is cooled below a body temperature, as described above. Therefore, the coiled stent 1 that supports the inner wall of an in vivo lumen from the inside while being placed in the in vivo lumen can be easily collected outside the body by hooking the hook 1b on a snare for collection 4 and pulling the hook 1b in the catheter 2.

The outside diameter of the stent 1 when it takes a coil form is preferably from 30 to 55 mm, approximately same as the inside diameter of the aorta that is an in vivo lumen. According to the preferable configuration, the stent 1 can put an appropriate tension to the inner wall of the blood vessel when it is placed in the aorta to treat aortic dissection. No tension is excessively put, so that the recovery of the entry part of aortic dissection can be properly promoted. Moreover, the outside diameter of the stent 1 when it takes a coil form preferably changes in a longitudinal direction. According to the preferable configuration, appropriate treatment can be offered because the outside diameter of the coil changes with changes in the inside diameter of the stenting site even if the inside diameter of the stenting site changes in a longitudinal direction. For example, the coil can firmly support an in vivo lumen when the inside diameter of an in vivo lumen is partially increased. Moreover, the stent does not put an excessive tension to an in vivo lumen even if the inside diameter of the in vivo lumen is partially decreased.

The diameter of the wire is preferably from 0.3 to 0.7 mm, particularly preferably from 0.4 to 0.6 mm. According to the preferable configuration, the stent 1 can efficiently control the formation of a blood clot while maintaining its strength. If the diameter of the wire is less than 0.3mm, the wire bits into the inner wall of an artery to damage. If the diameter of the wire exceeds 0.7m, the stent is hardly elongated and inserted in a catheter and hardly handled because, for example, the friction increases in the catheter.

The tensile strength of the wire is preferably from 900 to 1500 MPa, particularly preferably from 1000 to 1200 MPa. According to the preferable configuration, the stent 1 can put an appropriate tension to the inner wall of an in vivo lumen while maintaining its strength. If the tensile strength is less than 900 MPa, the dissociation part cannot be sufficiently recovered to its normal condition because the force to push the inner wall of an artery is weak. If the tensile strength exceeds 1500 MPa, an excessive tension may not shape the stent to suit a blood vessel or may not adjust the stenting position.

The material of the stent 1 is preferably any one of a nickel-titanium alloy, a stainless steel, titanium, and a titanium alloy. According to the preferable configuration, the stent 1 can be achieved by meeting the required biocompatibility and hardly making itself deformed, for example, crashed or folded even if it passes in a thin catheter 2. The stainless steel is preferable SUS304 or SUS316L. SUS304 is available and relatively inexpensive. SUS316L has the best corrosion resistance among in the stainless steels.

The wire preferably has a resin layer on its surface. According to the preferable configuration, the stent 1 can improve its biocompatibility. Moreover, the stent 1 can be appropriately frictioned against the inner wall of an in vivo lumen while it is delivered. In addition, the stent 1 can appropriately adjust its coil pitches on stenting. Therefore, the stent can sufficiently support the inner wall of an in vivo lumen from the inside.

The thickness of the resin layer is preferably from 0.01 to 3.00 mm. The resin layer with a thickness of 0.01 mm or more can reduce the possibility of damage to the stent because the thickness is not too thin. The resin layer with a thickness of 3.00 mm or less can efficiently control the formation of a blood clot because the thickness is not too thick.

The material of the resin layer is preferably any one of, for example, PU (polyurethane), PA (polyamide), PP (polypropylene), PE (polyethylene), and a fluorine resin. According to the preferable configuration, since it has biocompatibility, the stent 1 is not corroded to minimize the side effect to a human body after it has been inserted in an in vivo lumen. The resin layer is not limited to one type of material. For example, the entire wire may be covered with a resin such as PU (polyurethane) with a large frictional force against an in vivo lumen. Then, a fluorine resin that has a small frictional force against an in vivo lumen and hardly forms a blood clot may be coated on only the inside of the coil. This can easily place the stent 1 at an appropriate position in an in vivo lumen and efficiently control the formation of a blood clot.

The surface of the stent 1 is preferably coated with a medicine. According to the preferable configuration, the stent 1 can effectively promote the recovery of the treated area after stenting. The medicine is preferably a physiologically active substance, specifically a cell repairing agent, an anti-inflammatory agent, or an anticancer agent. Specifically, the medicine includes platelet inhibitors such as statin, rapamycin, aspirin, dipyridamole, heparin, an anti-thrombin agent, and fish oil; smooth-muscle proliferation depressants such as low-molecular-weight heparin and angiotensin converting enzyme inhibitor; anticancer agents such as vincristine sulfate, vinblastine sulfate, vindesine sulfate, irinotecan hydrochloride, paclitaxel, docetaxel hydrate, methotrexate, and cyclophosphamide; an antibacterial agent such as mitomycin C; immunosuppressants such as sirolimus and tacrolimus hydrate; an anti-inflammatory agent such as steroid; lipid-lowering agents such as atorvastatin calcium and lovastatin; plasmid DNA, a gene, siRNA, decoy type nucleic acid medicine (decoy), polynucleotide, oligonucleotide, antisense oligonucleotide, ribozyme, aptamer, interleukin, an intercellular messenger (cytokine), Glivec, and PTK787. However, the medicine coated on the surface of the stent 1 is not limited to these substances. Any one of the above-mentioned physiologically active substances may be coated. However, two or more kinds of components with different effects and mechanisms of action are coated to create synergy among their components, so that the promotion of the medicinal effect can be expected.

### Method of placing and collecting stent

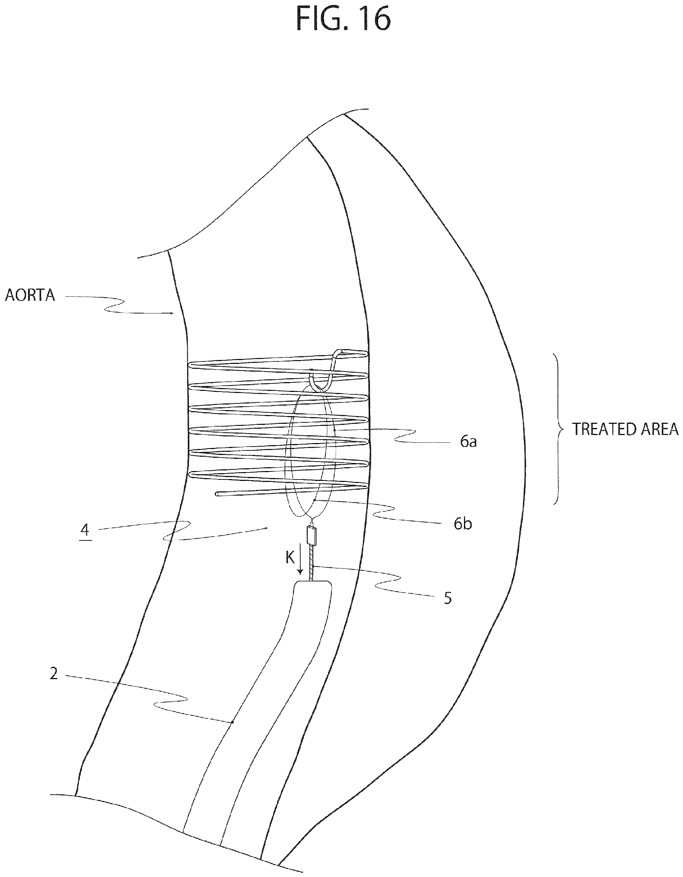

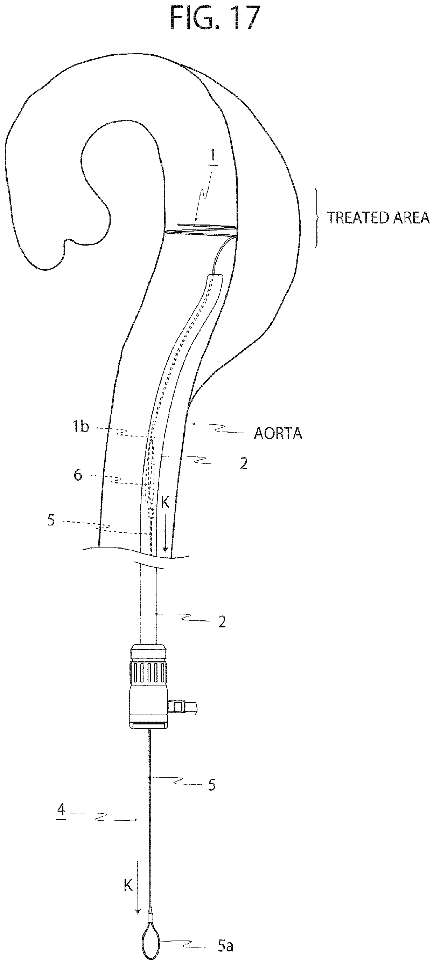

The method of placing the stent of Embodiment 1 of the present invention is explained below with reference to Figs. 4 to 18, giving an example where the stent used to treat aortic dissection is placed and collected.

Aorta has a three-layered structure including adventitia, media, and intima, which has enough strength and elasticity. However, an intimal tear (entry) occurs in the intima inside for some reasons, so that blood may flow into the media outside the intima to tear an aorta in a long axis direction (refer to the diagrams (a) and (b) of Fig. 4). This is called "aortic dissection." For a treatment of aortic dissection, temporally placing a stent inside a blood vessel is proposed (refer to the diagram (c) of Fig. 4).

### Method of placing stent

A stent is placed while being monitored by angiography. For stenting, the catheter introducer shown in Figs. 5 and 6 and a clamp for stent insertion (not shown) are used. The catheter introducer shown in Figs. 5 and 6 includes a catheter 2 that is a hollow flexible tube and a dilator 3 to be inserted in the catheter 2 from its bottom. When the dilator hub 3a is brought into contact with the bottom face of the catheter 2, the tip of the dilator 3 is projected from the tip of the catheter 2 (refer to Fig. 6). The clamp for stent insertion is used together with the catheter 2 that is a component of the catheter introducer.

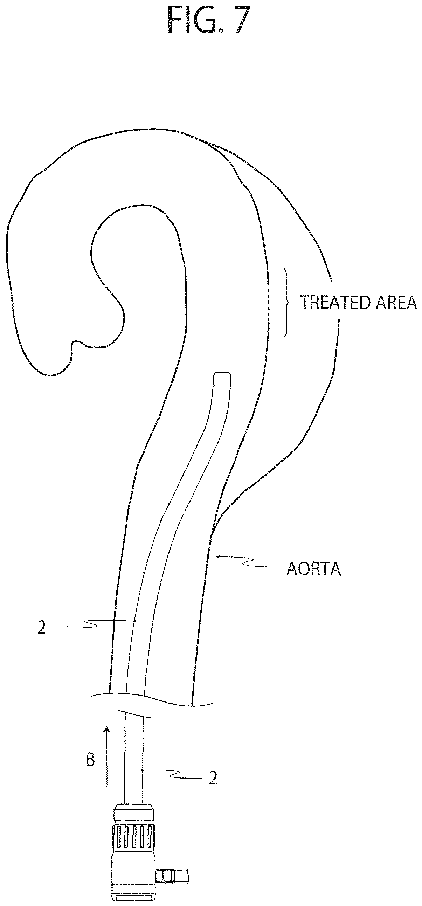

As shown in the diagram (a) of Fig. 4 and Fig. 7, a catheter 2 is first inserted in a blood vessel from the dissected base of a patient's leg (refer to the arrow B of Fig. 7), and the tip is brought to near the treated area in an aorta.

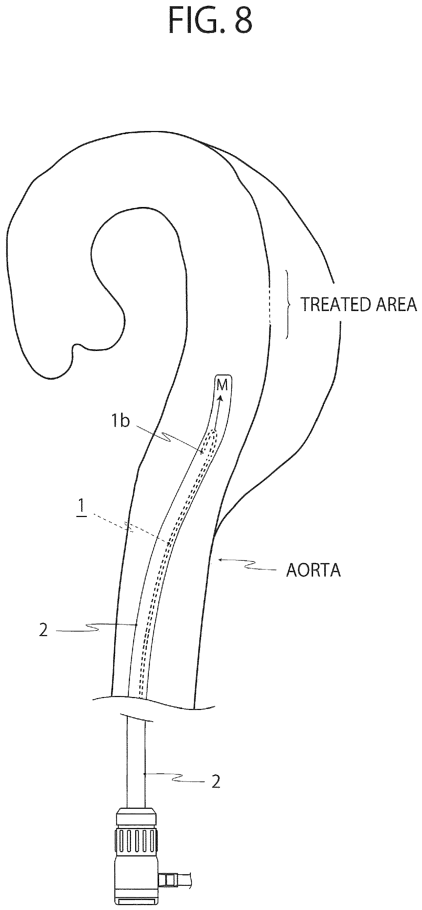

Next, as shown in Fig. 8, the bottom of the catheter 2 is held with the left hand, the grip part of the stent 1 (refer to Fig. 1) is gripped by a clamp for stent insertion held with the right hand, and then the stent 1 is inserted from the bottom face of the catheter 2. As the result, the stent 1 moves forward inside the catheter 2 (refer to the arrow M of Fig. 8), transforming itself from the coil form to the elongate form and reaches the tip of the catheter 2. When the stent 1 reaches the tip of the catheter 2, the clamp for stent insertion is removed from the catheter 2.

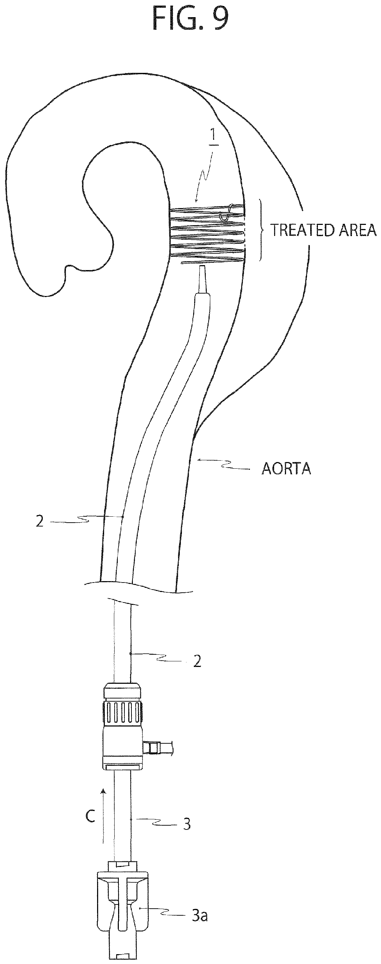

Next, as shown in Fig. 9, the bottom of the catheter 2 is held with the left hand, the dilator hub 3a is held with the right hand, and then the dilator 3 is inserted in the catheter 2 from its bottom (refer to the arrow C of Fig. 9). Subsequently, the catheter 2 is removed little by little while being rotated in the same direction as that the winding direction of the coiled stent 1, and then the stent 1 is pushed out of the tip of the catheter 2 with the dilator 3. At this time, the stent 1 is warmed to a body temperature and takes a coil form to support the inner wall of an in vivo lumen from the inside while being placed at a treated area in the in vivo lumen.

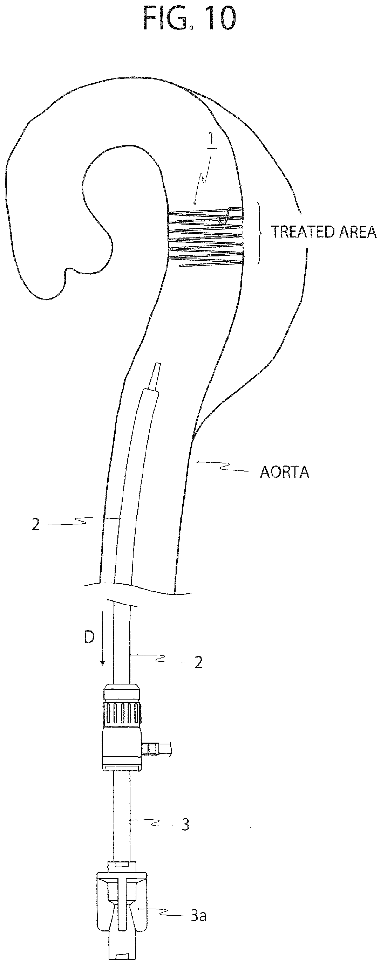

Finally, as shown in Fig. 10, the catheter introducer (the catheter 2 and the dilator 3) is removed from the dissected base of a patient's leg (refer to the arrow D of Fig. 10). The placement of the stent 1 used to treat aortic dissection is completed here.

### Method of collecting stent

After the treated area has been cured, the stent 1 that completed its role is collected as described below. The stent 1 is collected while being monitored by angiography. To collect the stent 1, the snare for collection 4 shown in Figs. 11 and 12 and the catheter 2 that is a hollow flexible tube are used.

The snare for collection 4 includes an operation wire 5 and a snare wire 6 as shown in Figs. 11 and 12. The operation wire 5 is inserted movably forward and backward inside a catheter that is a hollow flexible tube (refer to the double-headed arrow E of Fig. 12). An operation ring 5a is formed at the bottom of the operation wire 5. The operation ring 5a is held to move the operation wire 5 forward and backward (refer to the double-headed arrows E and F of Fig. 12) and rotate it inside the catheter 2. The snare wire 6 is provided at the tip of the operation wire 5. The snare wire 6 is formed of one wire and double looped in the absence of external forces, which includes a first loop 6a at the bottom side, a second loop 6b at the tip side, and an intersection 6c of the first loop 6a and the second loop 6b. The snare wire 6 is formed of a wire made of a shape-memory alloy. When the snare wire 6is pushed out of the tip of the catheter 2 in a lumen and warmed to a body temperature, the first loop 6a and the second loop 6b come close to each other and then are arranged approximately concentrically. The operation wire 5 is formed of a stranded wire that is produced by twisting multiple metallic wires such as stainless steel wires.

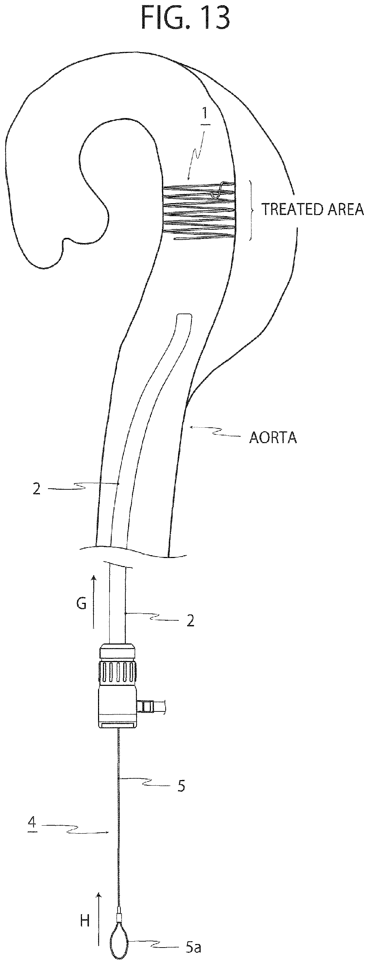

As shown in the diagram (a) of Fig. 4 and Fig. 13, after the snare for collection 4 is loaded inside of a catheter 2, the catheter 2 is first inserted in a blood vessel from the dissected base of a patient's leg (refer to the arrow G of Fig. 13), and the tip is brought to near the treated area in an aorta.

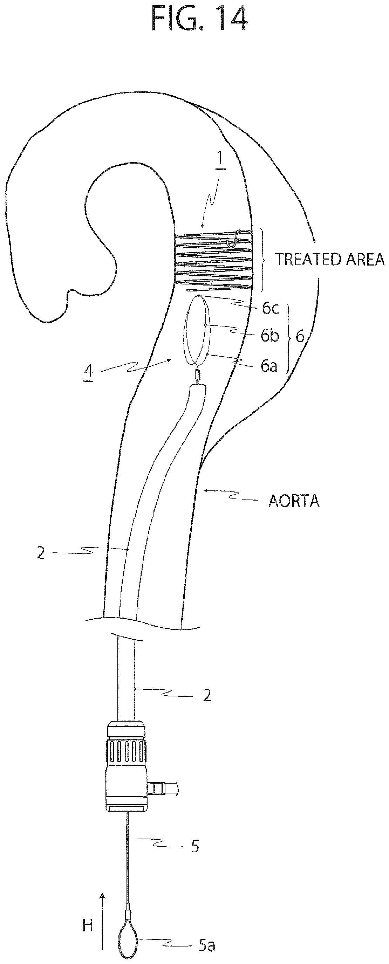

Next, the bottom of the catheter 2 is held with the left hand as shown in Figs. 13 and 14. The operation ring 5a at the bottom of the operation wire 5 is held with the right hand, and then the snare wire 6 is pushed out of the tip of the catheter 2 by moving the operation wire 5 forward (refer to the arrow H of Figs. 13 and 14). At this point, the snare wire 6 is warmed to a body temperature, and the first loop 6a and the second loop 6b come close to each other and then are arranged approximately concentrically.

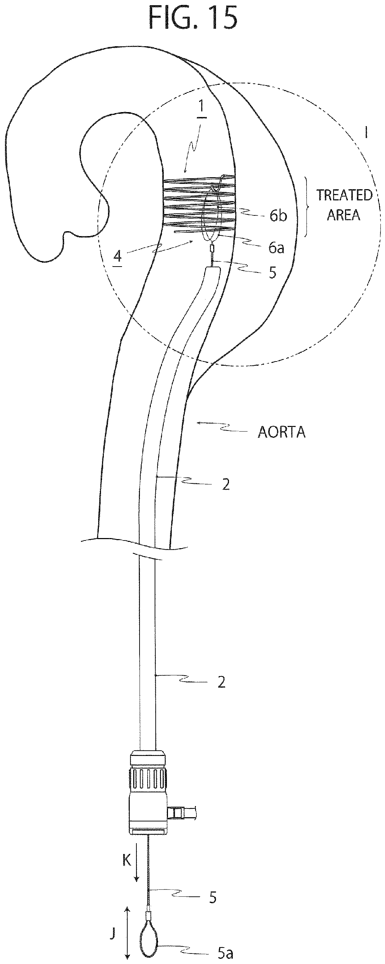

Next, as shown in Figs 14 to 16, the operation wire 5 is moved forward and backward (refer to the double-headed arrow J of Fig. 15) to hook the snare wire 6 on the hook 1b of the stent 1 (refer to Figs. 1 to 3). The snare wire 6 is formed of one wire and double looped, which includes a first loop 6a at the bottom side, a second loop 6b at the tip side, and an intersection 6c of the first loop 6a and the second loop 6b. The hook 1b of the stent 1 only has to be hooked by at least any one of the first loop 6a and the second loop 6b. Therefore, the snare wire 6 can be easily hooked by the hook 1b of the stent 1 without requiring any skills.

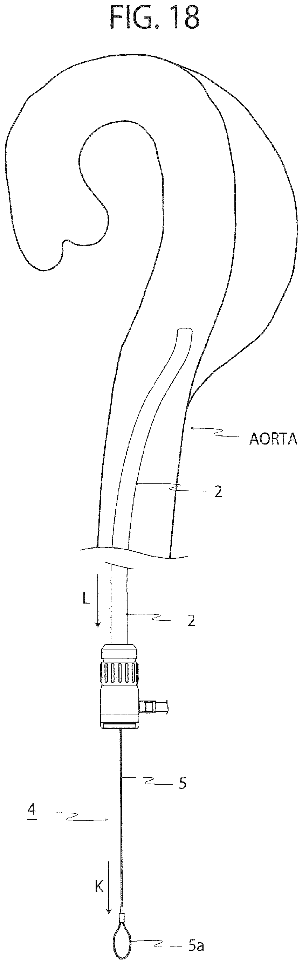

Next, the bottom of the catheter 2 is held with the left hand as shown in Figs. 15 to 18. The operation ring 5a at the bottom of the operation wire 5 moves the operation wire 5 backward (see the arrow K of Figs. 15 to 18) with the right hand to pull the stent 1 in the catheter 2. As the result, the stent 1 moves backward inside the catheter 2, transforming itself from the coil form to the elongate form and is collected outside the body. Finally, the catheter 2 is removed from the dissected base of a patient's leg (refer to the arrow L of Fig. 18). The collection of the stent 1 that was used to treat aortic dissection is completed here.

This embodiment has been explained, giving an example of the catheter 2 used that is a hollow flexible tube. However, the present invention is not necessarily limited to such an example. For example, a sheath can be used as the hollow flexible tube.

Moreover, this embodiment has been explained, giving an example of the stent 1 formed of a wire made of the shape-memory alloy. However, the present invention is not necessarily limited to such an example. For example, the stent of the present invention may be formed of a wire made of a superelastic alloy such as a stainless steel (SUS304) or a Ni-Ti alloy. The stent with this configuration is independent of temperature and takes an elongate form to elongate along the inside of the flexible tube by pulling the stent 1 in a longitudinal direction and a coil form to support the inner wall of an in vivo lumen from the inside by releasing the stent 1.

Moreover, this embodiment has been explained, giving an example of the stent 1 formed of a wire with a circular cross-section. However, the present invention is not necessarily limited to such an example. The wire may have a modified cross-section, in the same way as a flat wire.

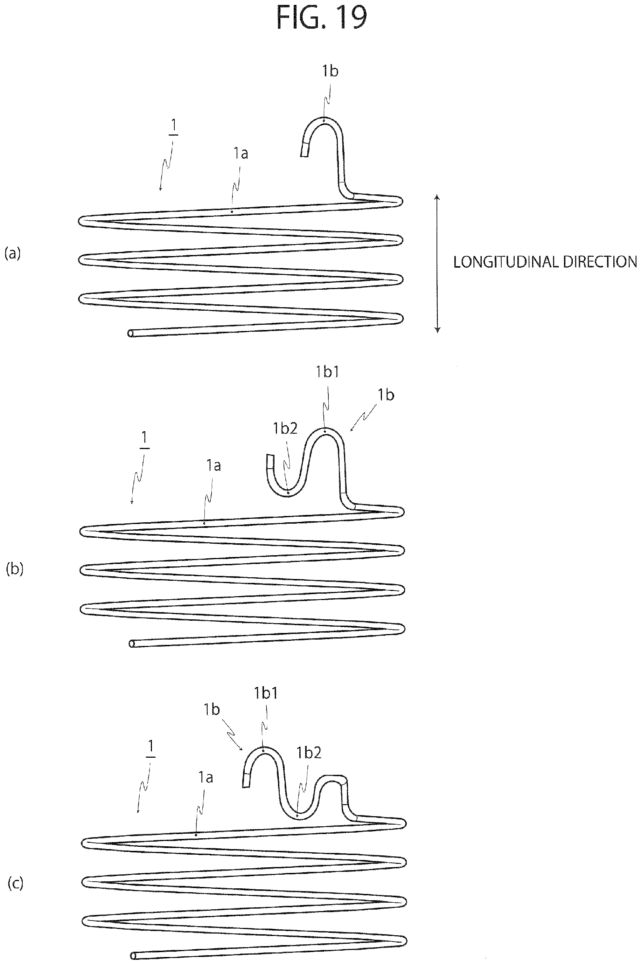

Moreover, this embodiment has been explained, giving an example where the hook 1b is located radially inside from the outer periphery of the stent main body 1a when the stent main body 1a takes a coil form to support the inner wall of an in vivo lumen from the inside. However, the present invention is not necessarily limited to such an example. As shown in the diagram (a) of Fig. 19, the hook 1b may project outside in a longitudinal direction when the stent main body 1a takes a coil form to support the inner wall of an in vivo lumen from the inside. Moreover, the hook 1b may have two bends 1b1 and 1b2 as shown in the diagrams (b) and (c) of Fig. 19. The two bends 1b1 and 1b2 bend in an opposite direction to each other in a longitudinal direction. The hook 1b with this configuration can easily collect the stent 1 from the thigh side and the collarbone side.

Moreover, this embodiment has been explained, giving an example of the stent 1 used to treat aortic dissection. However, the stent of the present invention is not necessarily limited to such an example. For example, the stent of the present invention can be inserted and placed in other blood vessels such as a coronary artery, a peripheral vessel, a carotid artery, a cerebral artery, and a vein, a trachea, a gullet, a large intestine, a small intestine, a duodenum, a ureter, a urethra, and a bile duct for its use.

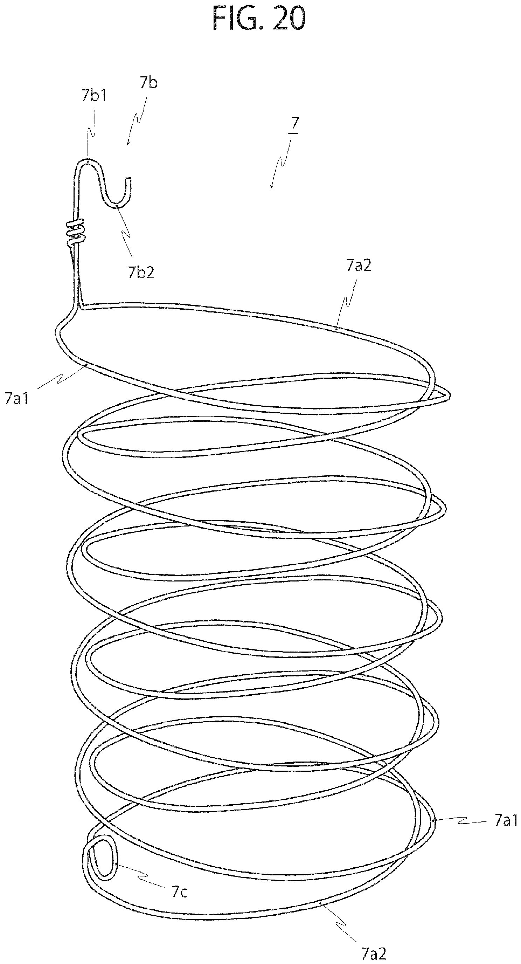

This embodiment has been explained, giving an example where the stent 1 has a hook 1b formed at its one end and is coiled from the hook 1b while maintaining the support form. However, the stent of the present invention is not necessarily limited to such an example. Other embodiments of the present invention will be described below with reference to Figs. 20 to 26.. In the embodiments described below, only the formation of the stent taking the support form is different from that of the above-mentioned Embodiment 1. The material, the diameter of wire, the methods of placing and collecting the stent are the same as those of the above-mentioned Embodiment 1. Therefore, the detailed explanation of these same matters is omitted.

### Embodiment 2

The configuration of the stent of Embodiment 2 of the present invention is described below with reference to Fig. 20.

The stent 7 shown in Fig. 20 is delivered from, for example, the dissected base of a patient's leg through the inside of a catheter 2 (refer to Figs. 5 and 6) that is a hollow flexible tube. The stent 7 is a support-formed member that supports the inner wall of an in vivo lumen from the inside while being placed at a treated area in the in vivo lumen (refer to Fig. 4). The stent 7 takes an elongate form to elongate along the inside of the catheter 2 by pulling the stent 7 in a longitudinal direction and a support form to support the inner wall of an in vivo lumen from the inside by releasing the tension in a longitudinal direction. The stent 7 also has a hook 7b for collection at its one end.

More specifically, the stent 7 of this embodiment has a hook 7b formed at its one end and also has a first helical part 7a1, a turned-back part 7c, and a second helical part 7a2 while maintaining the support form. The first helical part 7a1 elongates in a first helical from the hook 7b. The turned-back part 7c is formed at the end of the first helical part 7a1 that is opposite to the hook 7b. The second helical part 7a2 elongates in a second helical form from the turned-back part 7c and connects to the hook 7b. The second helical form is coiled in an opposite direction to the first helical form. The hook 7b has two bends 7b1 and 7b2 that bend in an opposite direction to each other. The stent 7 moves backward through the inside of the catheter 2, transforming itself from the support form to the elongate form, by hooking the hook 7b on a snare for collection 4 (refer to Figs. 11 and 12) and drawing the hook 7b through the inside of the catheter 2, and then is collected outside the body. Since it has two bends 7b1 and 7b2 that bend in an opposite direction to each other, the hook 7b can easily collect the stent 7 from the thigh side and the collarbone side.

Thus, according to the stent 7 of this embodiment, the stent 7 can sufficiently support the inner wall of an in vivo lumen from the inside and can be easily collected outside the body, in the same way as the stent 1 of Embodiment 1. The stent 7 of this embodiment can more evenly support the inner wall of an in vivo lumen from the inside by the two helical parts 7a1 and 7a2. This embodiment has been explained, giving an example where the stent 7 has a hook 7b provided with two bends 7b1 and 7b2 that bend in an opposite direction to each other. However, the present invention is not necessarily limited to such an example. The hook may bend in only one direction. The hook 1b may be located radially inside from the outer periphery of the stent 7 when the stent 7 takes a support form to support the inner wall of an in vivo lumen from the inside, in the same way as the stent of the above-mentioned Embodiment 1.

### Embodiment 3

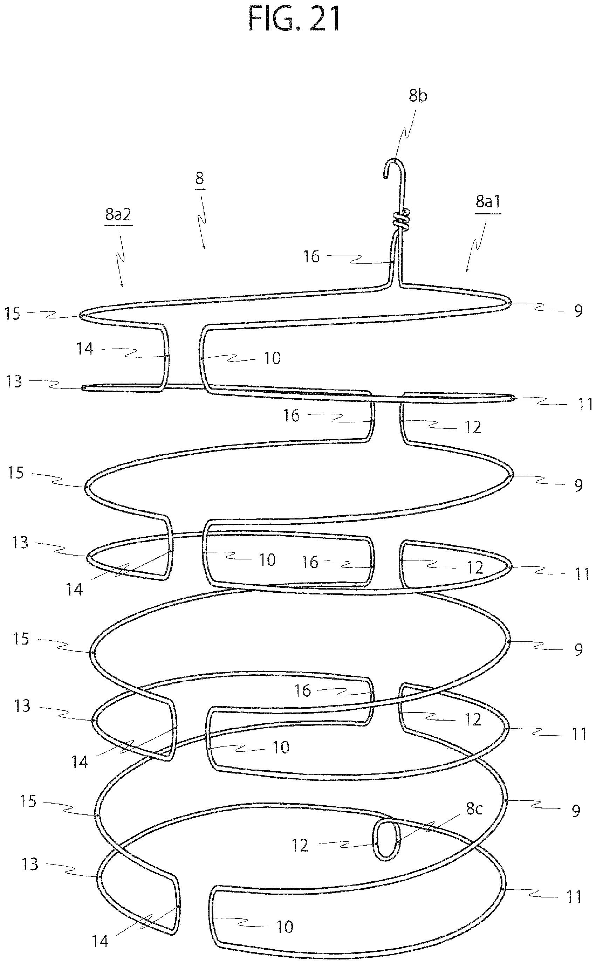

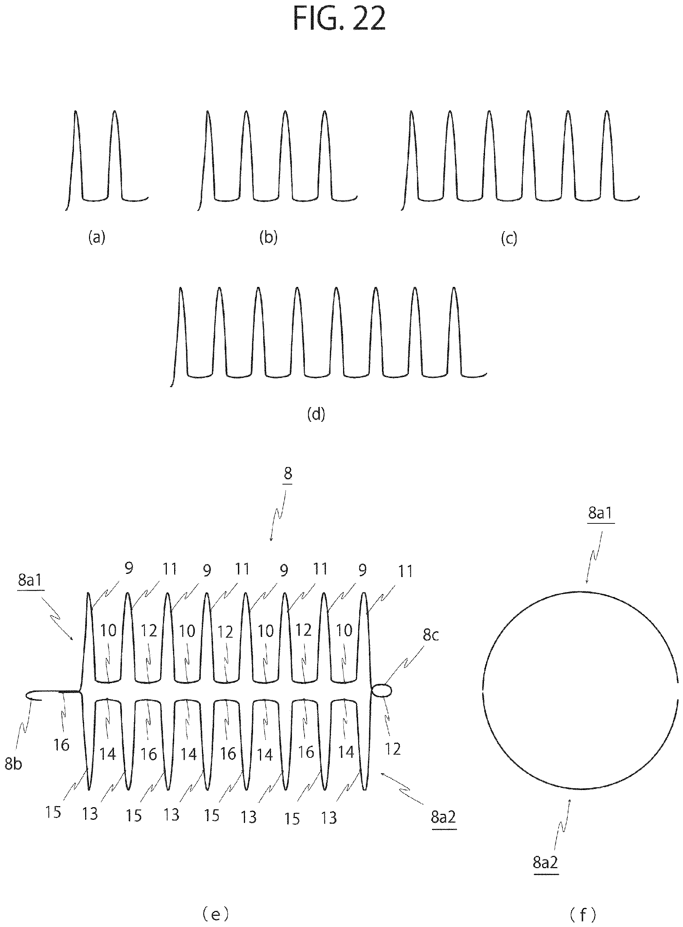

The configuration of the stent of Embodiment 3 of the present invention is described below with reference to Figs. 21 and 22.

The stent 8 shown in Fig. 21 and the diagrams (e) and (f) of Fig. 22 is delivered from, for example, the dissected base of a patient's leg through the inside of a catheter 2 (refer to Figs. 5 and 6) that is a hollow flexible tube. The stent 8 is a support-formed member that supports the inner wall of an in vivo lumen from the inside while being placed at a treated area in the in vivo lumen (refer to Fig. 4). The stent 8 takes an elongate form to elongate along the inside of the catheter 2 by pulling the stent 8 in a longitudinal direction and a support form to support the inner wall of an in vivo lumen from the inside by releasing the tension in a longitudinal direction. The stent 8 also has a hook 8b for collection at its one end.

More specifically, the stent 8 of this embodiment has a hook 8b formed at its one end and has a first pulse part 8a1, a turned-back part 8c, and a second pulse part 8a2 while maintaining the support form. The first pulse part 8a1 elongates in a first pulse form from the hook 8b in a longitudinal direction and is approximately semicircular, viewed from the end in a longitudinal direction. The turned-back part 8c is formed at the end of the first pulse part 8a1 that is opposite to the hook 8b. The second pulse part 8a2 elongates in a second pulse form from the turned-back part 8c toward the hook 8b and connects to the hook 8b. The second pulse form is plane-symmetrical against the first pulse form. The hook 8b bends in only one direction.

More specifically, the first pulse part 8a1 has four consecutive basic pulse forms each of which has a first semicircular part 9, a first longitudinal part 10, a second semicircular part 11, and a second longitudinal part 12. The first semicircular part 9 elongates in a semicircular form from the hook 8b in a circumferential direction. The first longitudinal part 10 elongates in a longitudinal direction from the end of the first semicircular part 9 that is opposite to the hook 8b. The second semicircular part 11 elongates in a semicircular form from the end of the first longitudinal part 10 that is opposite to the first semicircular part 9. The semicircular form is approximately parallel to the first semicircular part 9. The second longitudinal part 12 elongates in a longitudinal direction from the end of the second semicircular part 11 that is opposite to the first longitudinal part 10. More specifically, the second pulse part 8a2 has four consecutive basic pulse forms each of which has a third semicircular part 13, a third longitudinal part 14, a fourth semicircular part 15, and a fourth longitudinal part 16. The third semicircular part 13 elongates in a semicircular form from the turned-back part 8c. The semicircular form is line-symmetrical against the second semicircular part 11. The third longitudinal part 14 elongates approximately parallel to the first longitudinal part 10 from the end of the third semicircular part 13 that is opposite to the turned-back part 8c. The fourth semicircular part 15 elongates in a semicircular form from the end of the third longitudinal part 14 that is opposite to the third semicircular part 13. The semicircular form is approximately parallel to the first semicircular part 9. The fourth longitudinal part 16 elongates approximately parallel to the second longitudinal part 12 from the end of the fourth semicircular part 15 that is opposite to the third longitudinal part 14. The bends of the semicircular parts and the longitudinal parts are rounded R-shaped. Accordingly, the stent 8 does not damage the inner wall of an in vivo lumen during treatment. Moreover, the first and the second pulse forms may be various repeated forms as shown in the drawings (a) to (d) of Fig. 22.

The stent 8 moves backward through the inside of the catheter 2, transforming itself from the support form to the elongate form, by hooking the hook 8b on a snare for collection 4 (refer to Figs. 11 and 12) and drawing the hook 8b through the inside of the catheter 2, and then is collected outside the body.

The stent 8 of this embodiment more hardly falls over sideways in a longitudinal direction when or after it is placed at a treated area in an in vivo lumen, compared with the coiled stent. Moreover, the stent 8 hardly slides and moves over in an in vivo lumen because the wire forming the stent does not run in one direction. In addition, the stent 8 hardly crashes under pressure from the outer periphery because the semicircular parts of the wire forming the stent elongate in a circumferential direction and are parallel one after another. Therefore, the stent 8 can firmly support the inner wall of an in vivo lumen from the inside.

This embodiment has been explained, giving an example where the stent 8 has a hook 8b bending in only one direction. However, the present invention is not necessarily limited to such an example. For example, the hook 8b may be provided with two bends that bend in an opposite direction to each other in the same way as the above-mentioned Embodiment 2. Moreover, the hook 8b may be located radially inside from the outer periphery of the stent 8 when the stent 8 takes a support form to support the inner wall of an in vivo lumen from the inside, in the same way as the stent of the above-mentioned Embodiment 1.

### Embodiment 4

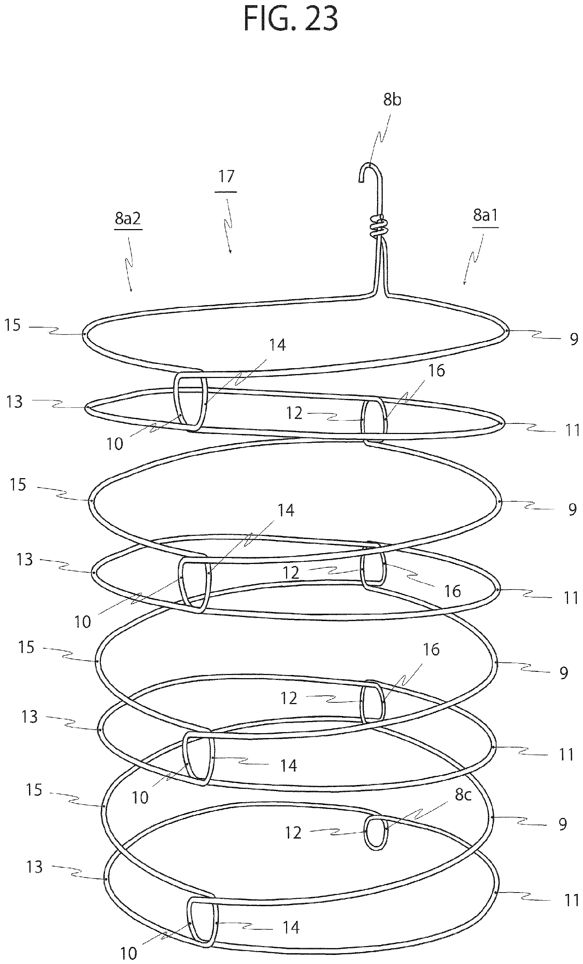

The configuration of the stent of Embodiment 4 of the present invention is described below with reference to Fig. 23. The configuration of the stent of this embodiment is the same as that of Embodiment 3 except that the pulse parts adjacent in a circumferential direction are interlaced and engaged with each other. Therefore, the same reference signs are used to refer the same members as those of Embodiment 3, and the detailed explanations are omitted.

In the stent 17 shown in Fig. 23, the pulse parts adjacent in a circumferential direction are interlaced and engaged with each other (interlacing engagement). Specifically, the first longitudinal part 10 and the third longitudinal part 14 that are adjacent to each other in a circumferential direction are interlaced and engaged with each other. Moreover, the second longitudinal part 12 and the fourth longitudinal part 16 that are adjacent to each other in a circumferential direction are interlaced and engaged with each other. This interlacing engagement can be achieved, for example, by interlacing the third longitudinal part 14 of the second pulse part 8a2 and the first longitudinal part 10 of the first pulse part 8a1 when the second pulse part 8a2 is bent.

The stent 17 of this embodiment can prevent the two pulse parts 8a1 and 8a2 from moving over in a longitudinal direction oppositely to each other to maintain its cylindrical form. Therefore, the stent 17 can more firmly support the inner wall of an in vivo lumen from the inside. The stent 17 can tightly support the inner wall of an in vivo lumen from the inside because the outside diameter of the stent can conform to the inside diameter of an in vivo lumen by not welding but interlacing and engaging the pulse parts adjacent in a circumferential direction with each other. Particularly when the inside diameter of an in vivo lumen is smaller than the outside diameter of the stent 17, the interlaced and engaged parts are biting into the inner wall of an in vivo lumen to put tension on the stent 17. Therefore, the stent can tightly support the inner wall of an in vivo lumen from the inside.

### Embodiment 5

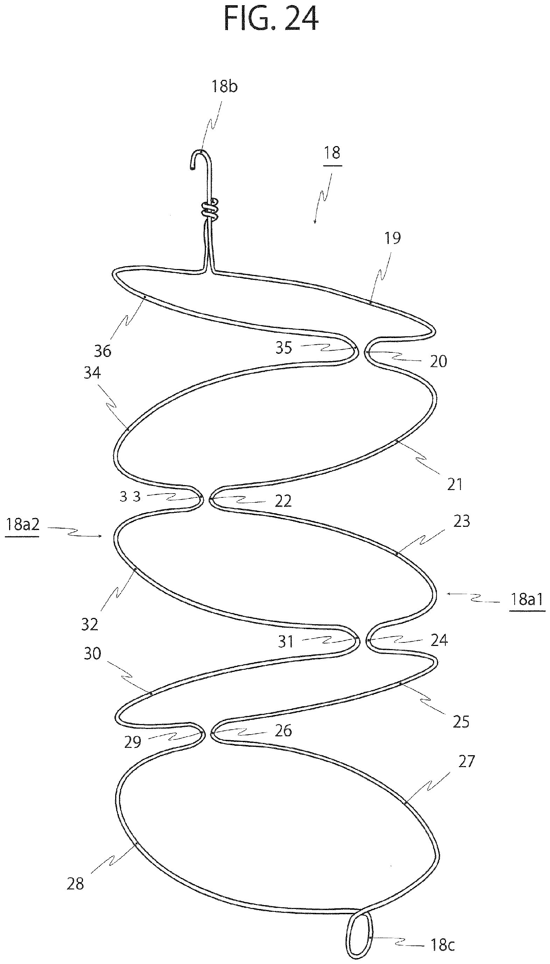

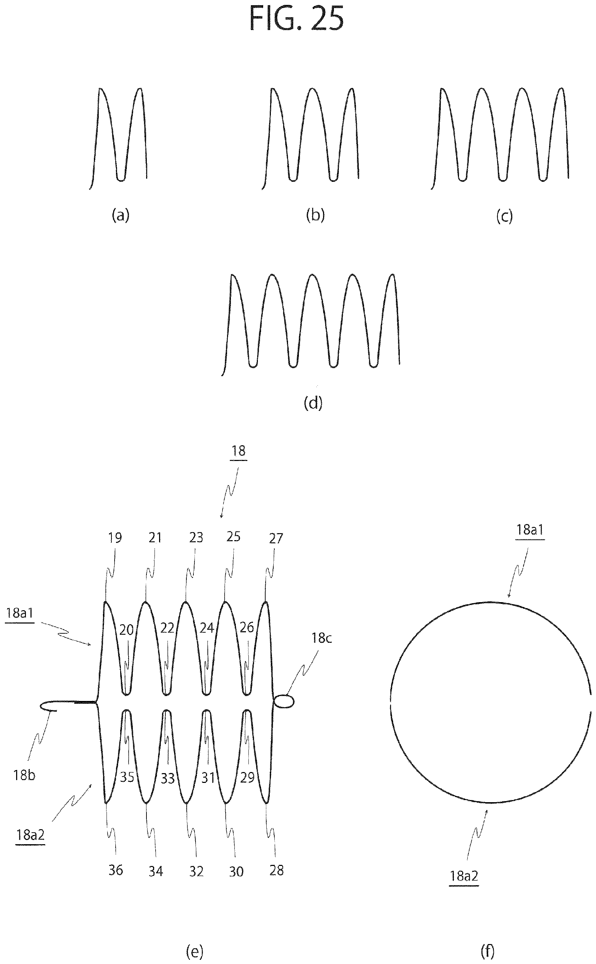

The configuration of the stent of Embodiment 5 of the present invention is described below with reference to Figs. 24 and 25.

The stent 18 shown in Fig. 24 and the diagrams (e) and (f) of Fig. 25 is delivered from, for example, the dissected base of a patient's leg through the inside of a catheter 2 (refer to Figs. 5 and 6) that is a hollow flexible tube. The stent 18 is a support-formed member that supports the inner wall of an in vivo lumen from the inside while being placed at a treated area in the in vivo lumen (refer to Fig. 4). The stent 18 takes an elongate form to elongate along the inside of the catheter 2 by pulling the stent 18 in a longitudinal direction and a support form to support the inner wall of an in vivo lumen from the inside by releasing the tension in a longitudinal direction. The stent 18 also has a hook 18b for collection at its one end.

More specifically, the stent 18 of this embodiment has a hook 18b formed at its one end and has a first zig-zag part 18a1, a turned-back part 18c, and a second zig-zag part 18a2 while maintaining the support form. The first zig-zag part 18a1 elongates in a first zig-zag form from the hook 18b in a longitudinal direction. The first zig-zag part 18a1 is approximately semicircular, viewed from the end in a longitudinal direction. The turned-back part 18c is formed at the end of the first zig-zag part 8a1 that is opposite to the hook 8b. The second zig-zag part 18a2 elongates in a second zig-zag form from the turned-back part 18c toward the hook 18b and connects to the hook 8b. The second zig-zag form is plane-symmetrical against the first zig-zag form. The hook 18b bends in only one direction.

More specifically, the first zig-zag part 18a1 has a first semicircular part 19, a first R-shaped part 20, a second semicircular part 21, a second R-shaped part 22, a third semicircular part 23, a third R-shaped part 24, a fourth semicircular part 25, a fourth R-shaped part 26, and a fifth semicircular part 27. The first semicircular part 19 elongates in a semicircular form from the hook 18b in a diagonally circumferential direction. The first R-shaped part 20 turns back from the end of the first semicircular part 19 that is opposite to the hook 18b. The second semicircular part 21 elongates in a semicircular form from the end of the first R-shaped part 20 that is opposite to the first semicircular part 19. The semicircular form is approximately plane-symmetrical against the first semicircular part 19. The second R-shaped part 22 turns back from the end of the second semicircular part 21 that is opposite to the first R-shaped part 20. The third semicircular part 23 elongates in a semicircular form from the end of the second R-shaped part 22 that is opposite to the second semicircular part 21. The semicircular form is approximately parallel to the first semicircular part 19. The third R-shaped part 24 turns back from the end of the third semicircular part 23 that is opposite to the second R-shaped part 22. The fourth semicircular part 25 elongates in a semicircular form from the end of the third R-shaped part 24 that is opposite to the third semicircular part 23. The semicircular form is approximately parallel to the second semicircular part 21. The fourth R-shaped part 26 turns back from the end of the fourth semicircular part 25 that is opposite to the third R-shaped part 24. The fifth semicircular part 27 elongates in a semicircular form from the end of the fourth R-shaped part 26 that is opposite to the fourth semicircular part 25. The semicircular form is approximately parallel to the third semicircular part 23.

More specifically, the second zig-zag part 18a2 has a sixth semicircular part 28, a sixth R-shaped part 29, a seventh semicircular part 30, a seventh R-shaped part 31, an eighth semicircular part 32, an eighth R-shaped part 33, a ninth semicircular part 34, a ninth R-shaped part 35, and a tenth semicircular part 36. The sixth semicircular part 28 elongates in a semicircular form from the turned-back part 18c. The semicircular form is approximately plane-symmetrical against the fifth semicircular part 27. The sixth R-shaped part 29 turns back from the end of the sixth semicircular part 28 that is opposite to the turn-back part 18c. The seventh semicircular part 30 elongates in a semicircular form from the end of the sixth R-shaped part 29 that is opposite to the sixth semicircular part 28. The semicircular form is approximately plane-symmetrical against the fourth semicircular part 25. The seventh R-shaped part 31 turns back from the end of the seventh semicircular part 30 that is opposite to the sixth R-shaped part 29. The eighth semicircular part 32 elongates in a semicircular form from the end of the seventh R-shaped part 31 that is opposite to the seventh semicircular part 30. The semicircular form is approximately plane-symmetrical against the third semicircular part 23. The eighth R-shaped part 33 turns back from the end of the eighth semicircular part 32 that is opposite to the seventh R-shaped part 31. The ninth semicircular part 34 elongates in a semicircular form from the end of the eighth R-shaped part 33 that is opposite to the eighth semicircular part 32. The semicircular form is approximately plane-symmetrical against the second semicircular part 21. The ninth R-shaped part 35 turns back from the end of the ninth semicircular part 34 that is opposite to the eighth R-shaped part 33. The tenth semicircular part 36 elongates in a semicircular form from the end of the ninth R-shaped part 35 that is opposite to the ninth semicircular part 34. The semicircular form is approximately plane-symmetrical against the first semicircular part 19.

The stent 18 of this embodiment is rounded overall. Accordingly, the stent 18 does not damage the inner wall of an in vivo lumen during treatment. Moreover, the first and the second zig-zag forms may be various repeated forms as shown in the drawings (a) to (d) of Fig. 25.

The stent 18 moves backward through the inside of the catheter 2, transforming itself from the support form to the elongate form, by hooking the hook 18b on a snare for collection 4 (refer to Figs. 11 and 12) and drawing the hook 7b through the inside of the catheter 2, and then is collected outside the body.

The stent 18 of this embodiment more hardly falls over sideways in a longitudinal direction when or after it is placed at a treated area in an in vivo lumen, compared with the coiled stent. Moreover, the stent 18 hardly slides and moves over in an in vivo lumen because the wire forming the stent does not run in one direction. In addition, since it elongates in a zig-zag form in a longitudinal direction, the stent 18 more hardly falls over sideways, compared with the stent 8 of the above-mentioned Embodiment 3. Therefore, the stent 18 can firmly support the inner wall of an in vivo lumen from the inside.

This embodiment has been explained, giving an example where the stent has a hook 18b bending in only one direction. However, the present invention is not necessarily limited to such an example. For example, the hook 18b may be provided with two bends that bend in an opposite direction to each other in the same way as the above-mentioned Embodiment 2. Moreover, the hook 18b may be located radially inside from the outer periphery of the stent 18 when the stent 18 takes a support form to support the inner wall of an in vivo lumen from the inside, in the same way as the stent of the above-mentioned Embodiment 1.

### Embodiment 6

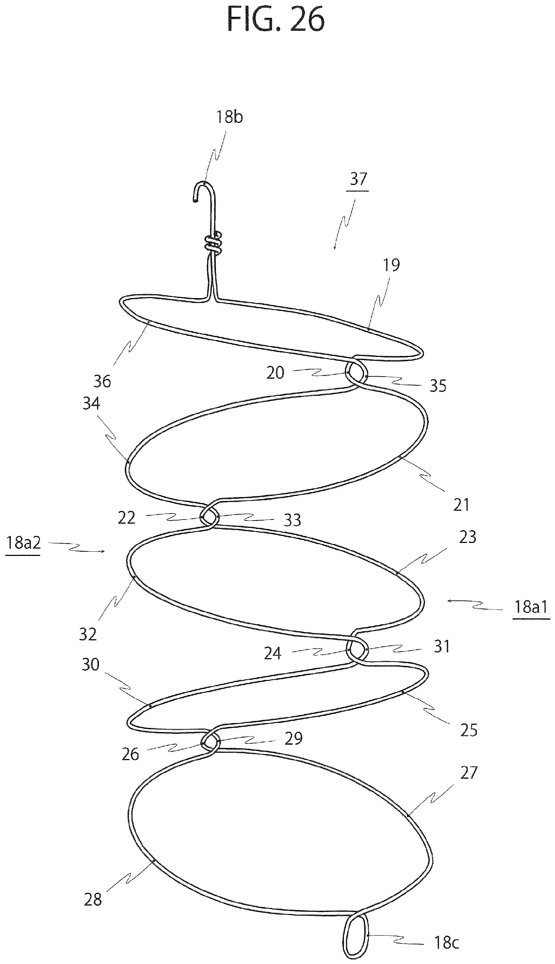

The configuration of the stent of Embodiment 6 of the present invention is described below with reference to Fig. 26. The configuration of the stent of this embodiment is the same as that of Embodiment 5 except that the zig-zag parts adjacent in a circumferential direction are interlaced and engaged with each other. Therefore, the same reference signs are used to refer the same members as those of Embodiment 5, and the detailed explanations are omitted.

In the stent 37 shown in Fig. 26, the adjacent zig-zag parts are interlaced and engaged with each other in a circumferential direction (interlacing engagement). Specifically, the first R-shaped part 20 and the ninth R-shaped part 35 that are adjacent to each other in a circumferential direction are interlaced and engaged with each other. Moreover, the second R-shaped part 22 and the eighth R-shaped part 33 that are adjacent to each other in a circumferential direction are interlaced and engaged with each other. Moreover, the third R-shaped part 24 and the seventh R-shaped part 31 that are adjacent to each other in a circumferential direction are interlaced and engaged with each other. Moreover, the fourth R-shaped part 26 and the sixth R-shaped part 29 that are adjacent to each other in a circumferential direction are interlaced and engaged with each other. This interlacing engagement can be achieved, for example, by interlacing the sixth R-shaped part 29, the seventh R-shaped part 31, the eighth R-shaped part 33, and the ninth R-shaped part 35 of the second zig-zag part 18a2 and the fourth R-shaped part 26, the third R-shaped part 24, the second R-shaped part 22, and the first R-shaped part 20 of the first zig-zag part 18a1, respectively in this order, when the second pulse part 18a2 is bent.

The stent 37 of this embodiment can prevent the two zig-zag parts 18a1 and 18a2 from moving over in a longitudinal direction oppositely to each other to maintain its cylindrical form. Therefore, the stent 37 can more firmly support the inner wall of an in vivo lumen from the inside. The stent 37 can tightly support the inner wall of an in vivo lumen from the inside because the outside diameter of the stent can conform to the inside diameter of an in vivo lumen by the adjacent zig-zag parts interlaced and engaged with each other in a circumferential direction. Particularly when the inside diameter of an in vivo lumen is smaller than the outside diameter of the stent 37, the interlaced and engaged parts are biting into the inner wall of an in vivo lumen to put tension on the stent 37. Therefore, the stent can tightly support the inner wall of an in vivo lumen from the inside.

### DESCRIPTION OF REFERENCE NUMBERS:

1, 7, 8, 17, 18, 37-Stent;
1a-Stent main body;
1b, 7b, 8b, 18b-Hook;
2-Catheter;
4-Snare for collection;
7a1-First helical part;
7a2-Second helical part;
7c, 8c, 18c-Turned-back part;
8a1-First pulse part;
8a2-Second pulse part;
18a1-First zig-zag part;
18a2-Second zig-zag part;

## Claims

1. A stent that is delivered through the inside of a flexible hollow tube and supporting the inner wall of an in vivo lumen from the inside while the stent is being placed in the in vivo lumen, comprising:
a structure that takes:
an elongate form to elongate along the inside of the flexible tube by pulling the stent in a longitudinal direction, and
a support form to support the inner wall of the in vivo lumen from the inside by releasing the stent; and
a hook for collection, wherein
the stent moves backward through the inside of the flexible tube, transforming itself from the support form to the elongate form, by hooking the hook on a snare for collection and pulling the hook in the flexible tube, and then the stent is collected outside the body.

2. The stent according to claim 1, wherein the hook is formed at one end of the stent, and the stent is coiled from the hook while maintaining the support form.

3. The stent according to claim 1,
wherein the hook is formed at one end of the stent, and
the stent while maintaining the support form comprises:
a first helical part that elongates in a first helical form from the hook;
a turned-back part that is formed at the end of the first helical part that is opposite to the hook; and
a second helical part that elongates in a second helical form from the turned-back part and connects to the hook, the second helical form that is coiled in an opposite direction to the first helical form.

4. The stent according to claim 1, wherein
the hook is formed at one end of the stent, and
the stent while maintaining the support form comprises:
a first pulse part that elongates in a first pulse form from the hook in a longitudinal direction, the first pulse part that is approximately semicircular, viewed from the end in a longitudinal direction;
a turned-back part that is formed at the end of the first pulse part that is opposite to the hook; and
a second pulse part that elongates in a second pulse form from the turned-back part toward the hook and connects to the hook, the second pulse form that is plane-symmetrical against the first pulse form.

5. The stent according to claim 4, wherein the pulse parts adjacent in a circumferential direction are interlaced and engaged with each other.

6. The stent according to claim 1, wherein the hook is formed at one end of the stent, and
the stent while maintaining the support form comprises:
a first zig-zag part that elongates in a first zig-zag form from the hook in a longitudinal direction, the first zig-zag part that is approximately semicircular, viewed from the end in a longitudinal direction;
a turned-back part that is formed at the end of the first zig-zag part that is opposite to the hook; and
a second zig-zag part that elongates in a second zig-zag form from the turned-back part toward the hook and connects to the hook, the second zig-zag form that is plane-symmetrical against the first zig-zag form.

7. The stent according to claim 6, wherein the zig-zag parts adjacent in a circumferential direction are interlaced and engaged with each other.

8. The stent according to any one of claims 1 to 7, the stent that is formed of a shape-memory alloy wire, comprising a structure that takes an elongate form to elongate along the inside of the flexible tube when the stent is cooled below a body temperature, and a support form to support the inner wall of an in vivo lumen from the inside when the stent is warmed to a body temperature.

9. The stent according to any one of claims 1 to 8, wherein the hook is located in a radially inner direction from the outer periphery of the stent when it takes the support form.

10. The stent according to any one of claims 1 to 9, wherein the outside diameter of the stent when it takes the support form is from 30 to 55 mm, approximately same as the inside diameter of the aorta that is the in vivo lumen.

11. The stent according to claim 10, wherein the outside diameter of the stent changes in a longitudinal direction when it takes the support form.

12. The stent according to any one of claims 8 to 11, wherein the diameter of the wire is from 0.3 to 0.7 mm.

13. The stent according to any one of claims 8 to 12, wherein the tensile strength of the wire is from 900 to 1500 MPa.

14. The stent according to any one of claims 8 to 13, wherein the material of the wire is any one of a nickel-titanium alloy, a stainless steel, titanium, and a titanium alloy.

15. The stent according to any one of claims 8 to 14, wherein the wire has a resin layer on the surface.

16. The stent according to claim 15, wherein the thickness of the resin layer is from 0.01 to 3.00 mm.

17. The stent according to claim 15 or 16, wherein the material of the resin layer is preferably any one of PU (polyurethane), PA (polyamide), PP (polypropylene), PE (polyethylene), and a fluorine resin.

18. The stent according to any one of claims 1 to 17, wherein the surface of the stent is coated with a medicine.

19. The stent according to claim 18, wherein the medicine is a physiologically active substance.

20. The stent according to any one of claims 1 to 19, wherein the stent is used to treat aortic dissection.
